# EUROPEAN PATENT SPECIFICATION

(11) **EP 3 749 258 B1**
(45) Date of publication and mention of the grant of the patent: **07.01.2026**
(21) Application number: 18797200.5
(22) Date of filing: 07.02.2018
(51) Int. Cl.: A61F 2/58, B25J 15/00, B25J 15/02, B25J 15/08, B25J 15/10, A61F 2/68

(54) **A PROSTHETIC HAND**
PROTHESENHAND
PROTHÈSE DE MAIN

(43) Date of publication of application: 16.12.2020
(73) Proprietor: KOC Universitesi, 34450 Istanbul (TR)
(72) Inventor: LAZOGLU, Ismail, 34450 Istanbul (TR); SHAMS, Sarmad, 34450 Istanbul (TR)
(74) Representative: Atalay, Baris
(86) International application number: PCT/TR2018/050048
(87) International publication number: WO 2019/156643

(56) References cited:
- WO-A1-2012/039479
- WO-A1-2014/027897
- US-A1- 2007 035 143
- US-A1- 2016 074 181
- US-A1- 2017 266 019

## Description

### Technical Field of the Present Invention

The invention presented hereby generally concerns a prosthetic hand with thumb and finger members, to be used by amputees or robots in order to perform most of the daily life activity grip and hold actions. The invention specifically concerns a combination of a finger slider as a linear actuator driving a proximal phalange at a connecting link, said proximal phalange member being configured as a meeting point between a conjoined four-bar mechanism and a slider-crank mechanism to achieve extension for finger members of a prosthetic hand.

### Prior Art/Background of the Present Invention

An artificial/prosthetic hand is a device used by amputees and robots alike as a terminal device to interact with the surroundings by touching, gripping, holding, picking and placing various surfaces and objects. During a gripping process the fingers should be able to either adopt the shape of the object being grasped (in case of small objects), or hold the object with effectively firm gripping mechanism, so that the object does not fall off the hand, the need for the robustness of finger mechanism notwithstanding.

Today, most of the prosthetic hands use electric motors for the actuation of individual fingers. However, the size of an electric motor is not small enough for designs to include motors at joints to actuate the fingers. Instead, most designs have to devise a mechanism to translate the rotation of the motor into flexion and extension of fingers via secondary mechanisms such as cables, tendons and worm gears. Present invention offers a mechanism for the movement of individual fingers by uniquely integrating a four-bar mechanism with a slider crank mechanism. The slider in the slider crank mechanism either actuated by a linear actuator or by lead-screw mechanism driven by a motor or any other possible actuation method. For the thumb, slider crank mechanisms are utilized both for actuating the thumb and for promoting effective opposability.

Previous technique includes different implementations of such methods. One such prior art example; US2017049583A1 includes a cable-driven hand capable of multiple grasp types in the case of which actuation of individual fingers are handled with bar-connected tendon lines. Thumb possesses its individual orientation cable which enables lockability into alignment with the index finger, enabling pinch. Body-powered cable induces position variations between fingers for power and precision grasps.

Another patent application US2015216681A1 publication discloses a myoelectric prosthetic hand comprising a rotor-motor and transmission element actuating at least one finger with the aid of a linkage. Furthermore, it includes a clutch for non-back drivability i.e. prevention of output motion to be transmitted, a separately rotatable wrist which has its designated transmission.

Publication number US2016250044A1 discloses another prosthetic hand with multi-level gear locking mechanism comprising a first internal gear attached to a distal finger link, a second internal gear attached to a proximal finger link and an external gear coupled to a button wherein the external gear forms a bridge between the first internal gear and the second internal gear to lock a finger joint of the finger member, along with a spring to retain the position.

A prosthetic hand with the publication number WO2017111582A1 discloses such, characterized with finger members attached to a palm of hand with said finger members comprising bars and joints connecting bars to form a construction supported by a wrist. Fingers can possess a plurality of loaded positions depending on the load applied to the inner side of the hand, the opposite condition of which includes the finger members to close the hand and a locking mechanism locks the construction in one of the plurality of the loaded positions.

A prior art document identified by the publication number WO2012/039479 discloses a humanoid electric hand comprising a finger motor for driving a finger is linked to a worm reduction mechanism. A worm wheel as the output gear of the worm reduction mechanism rotates, so that a metacarpophalangeal joint bends and extends. The metacarpophalangeal joint is linked through two link mechanisms consisting of a first driving link and a second driving link to a proximal interphalangeal joint and a distal interphalangeal joint. This allows the proximal interphalangeal joint and the distal interphalangeal joint to bend and extend in conjunction with the bending and extending operations of the metacarpophalangeal joint.

Another publication with the reference US2017/266019 discloses a control unit of a limb device for electrically controlling an electrically controllable limb device comprising a plurality of actuators. The control unit comprises a first interface for connecting the control unit to the limb device, a second interface for connecting the control unit to a data gathering device comprising one or more sensing devices, a processing unit which is arranged for controlling the limb device at least based on data gathered by the data gathering device, wherein the control unit is arranged for outputting one single control action step to the actuators of the limb device calculated by the processing unit based on a first data or data combination received from the data gathering device. The control unit is also arranged for outputting a plurality of control action steps to the actuators of the limb device calculated by the processing unit based on a second data or data combination received from the data gathering device, the second data or data combination being different from the first data or data combination, the plurality of control action steps inducing a more complex automatic movement of the limb device that the one single control action step.

US2016/074181 discloses Systems and methods for postural control of a multi-function prosthesis using EMG signals to drive a point in a posture space and outputting continuously varying joint angles for a powered prosthetic hand. The postural controller can include an EMG signal processing unit to receive signals from electrodes for processing. The processed EMG signals can then be combined or converted to produce a point in the postural control domain. The PC domain map defines the posture that corresponds to each PC cursor coordinate. This map can have limitless possible postures and limitless possible positions of the postures. The Joint Angle Transform converts the PC cursor coordinate into the joint angle array which is sent to the prosthetic hand thereby creating more natural movements.

US2007/035143 discloses a robotic hand and arm where the fingers are driven by rotational motors with drums, and pulled with cables using rolling friction. The hand extends into a robotic arm through a wrist controlled by pneumatic cylinders. Each finger preferably is provided with four manufactured parts and a single pulley. The thumb is provided with three pulleys for independent distal movement. Cables wraps around or over pulleys, eliminating tight bends. A glove is provided about the robotic hand which provides a compressive, liquid resistant membrane.

WO2014/027897 discloses a gripping device such as an automated hand, comprising a plurality of metacarpal members, each metacarpal member having a first end attached to a support frame via a universal joint and having a second end attached to a gripping member, wherein the metacarpal members are able to pivot upwardly, downwardly and laterally. Where adjacent metacarpal members together form a palm of the hand, the ability of the metacarpal members to move up, down and laterally helps the palm to grip an object and also helps the palm to withstand at least some forces from above, below and from the side.

### Objects of the Present Invention

Primary object of the present invention is to provide a prosthetic hand to be used by amputees and robots alike, that is capable of different styles of gripping and holding motions.

Another object of the present invention is to integrate a grip mechanism that mimics the lifelike application of said styles of gripping and holding positions of the person to wield the prosthetic hand disclosed in the present invention having a transradial or transhumeral amputation, with the aid of a user trained state machine.

### Summary of the Present Invention

A prosthetic hand employs the principle of an analogous structure regarding a human hand which targets the adequate and efficient execution of hand-oriented tasks such as holding and grasping with a multiplicity of finger members and an opposable thumb.

An artificial or prosthetic hand continuously interacts with the environment either grasping an object, carrying different objects, reaching places that human beings cannot, among many others. During this interaction, continuous monitoring of the force as feedback is crucial to perform delicate and sophisticated tasks like holding an egg, handshaking with a person or holding a paper cup with a critical level of liquid contained within. In all cases, it is required to apply an appropriate amount of force so that the egg does not collapse, hand is not squeezed because of a force undue or paper cup does not fall and retains a condition so that the liquid is drinkable. To monitor interaction force, a feedback or contact force sensor exists that detects the force by using different phenomena e.g. piezoresistivity, conductive or inductive, piezoelectric and strain gauges.

Multiplicity of finger members in the present invention also present a high level of dexterity for adequate replication of actions a hand is capable of performing. The relative position of finger and thumb members grant the user, be it a human or a robot, with a proficiency in pinch and lateral-grip of items like cards, power grip of items such as mobile phones, and index finger pointing.

Disclosed invention also seeks to address the cost and weight problems of many prostheses with its material choice and design that includes allocation of the heavier elements towards parts of the bodies to induce less torque on the shoulder, with the utilization of a forearm chamber in relation with the humerus and the upper arm.

This is achieved according to the present disclosure by providing a multiplicity of finger members, said finger members each comprising a finger connecting link having a transmitting link in the form of motion with conjoined four-bar and slider-crank mechanisms. Said link is connected to the actuator in a metacarpal member provided for finger members using a finger slider delivering linear motion for actuation. Said finger slider is configuted to be a linear actuator which drives a proximal phalange member of said finger members for extension, forwards and backwards, at the connecting link. By this, a transmitting link at the connecting link constituting conjoined four-bar and slider-crank mechanisms is driven, wherein said proximal phalange member is configured as a meeting point between said conjoined four-bar and slider-crank mechanisms.

Furtner, disclosed invention also comprises a thumb member opposability of which is ensured by an opposability actuator in a palm member, said opposability actuator is structurally associated with a base of said thumb with an opposability connecting link.

### Brief Description of the Figures of the Present Invention

Accompanying drawings are given solely for the purpose of exemplifying a prosthetic hand, whose advantages over prior art were outlined above and will be explained in brief hereinafter.

The drawings are not meant to delimit the scope of protection as identified in the claims nor should they be referred to alone in an effort to interpret the scope identified in said claims without recourse to the technical disclosure in the description of the present invention.

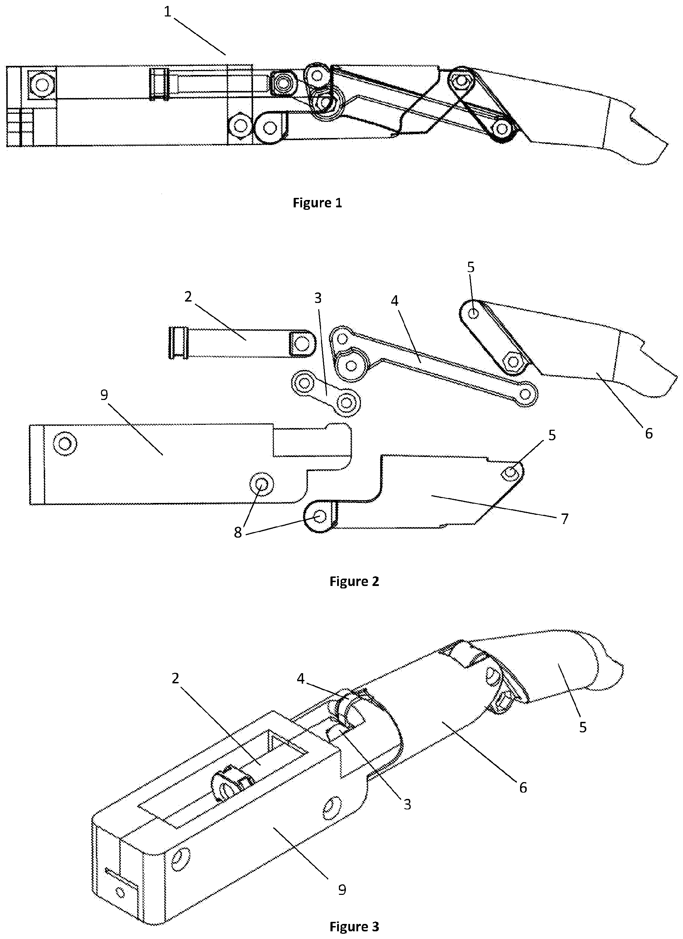

Fig. 1 demonstrates side view of a finger member according to the present invention.
Fig. 2 demonstrates exploded side view of a finger assembly according to the present invention.
Fig. 3 demonstrates isometric view of a finger assembly according to the present invention.

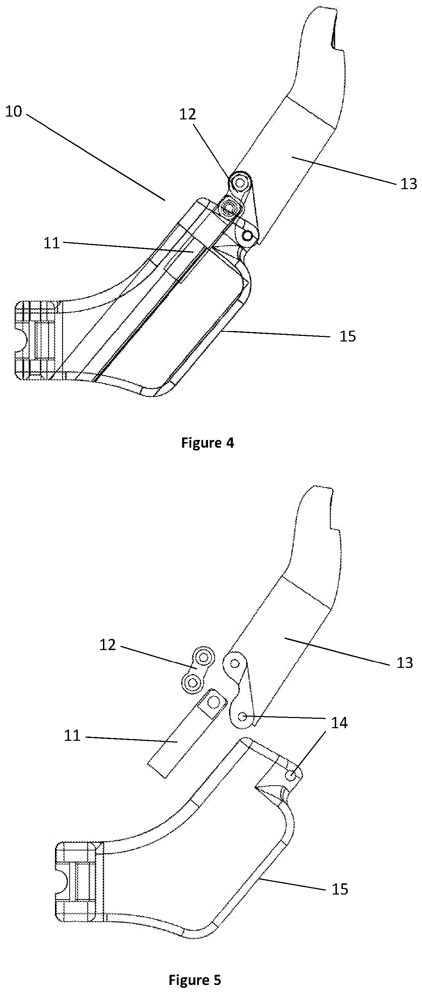

Fig. 4 demonstrates side view of a thumb assembly according to the present invention.
Fig. 5 demonstrates exploded side view of a thumb assembly according to the present invention.

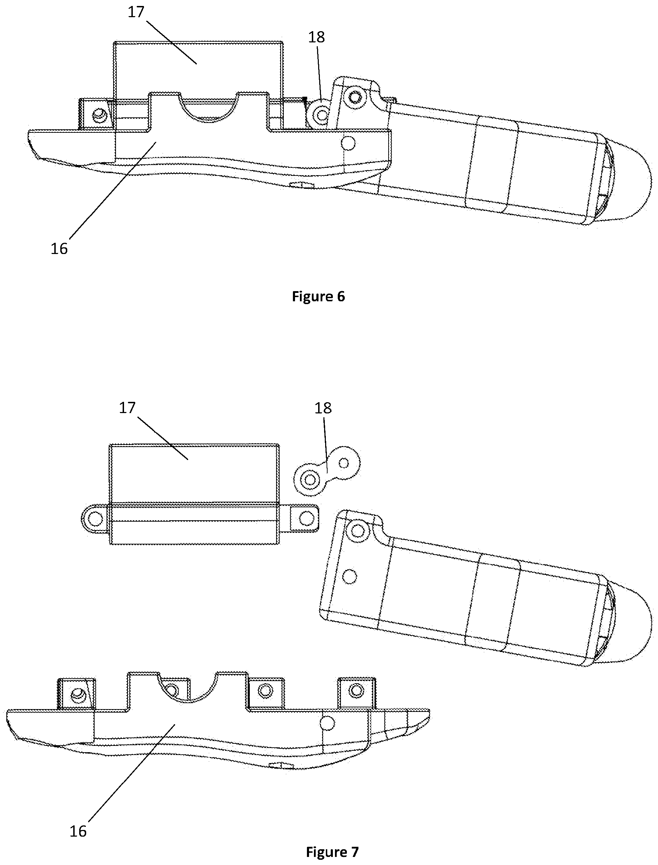

Fig. 6 demonstrates thumb assembly, thumb actuator and palm looking in from the wrist joint according to the present invention.
Fig. 7 demonstrates exploded view of thumb assembly, thumb actuator and palm looking in from the wrist joint according to the present invention.

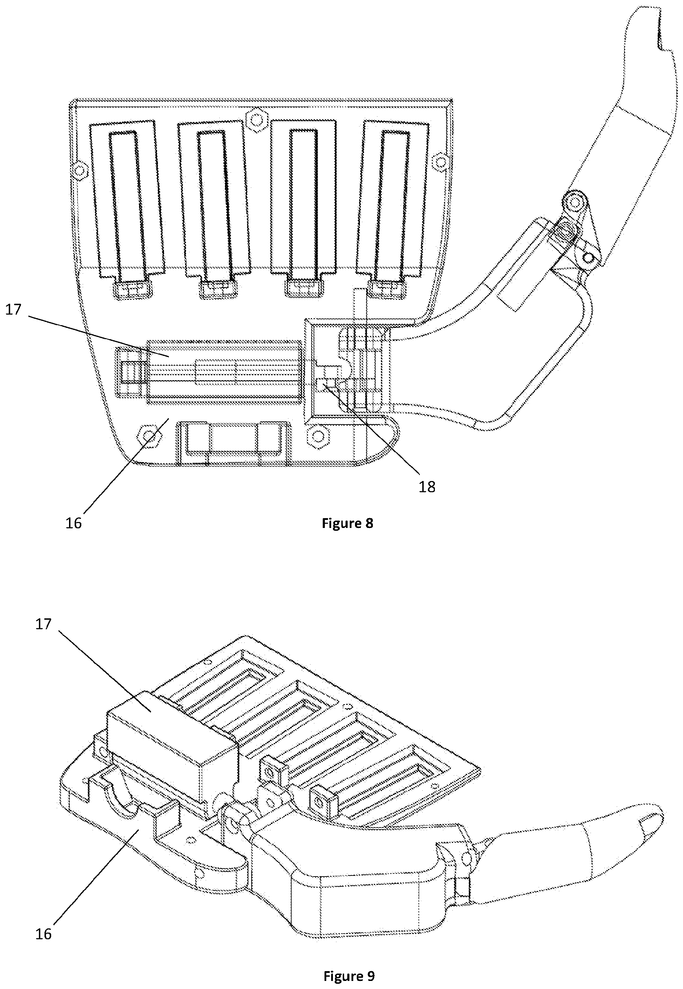

Fig. 8 demonstrates top view of thumb assembly, thumb actuator and palm according to the present invention.
Fig. 9 demonstrates isometric view of thumb assembly, thumb actuator and palm according to the present invention.

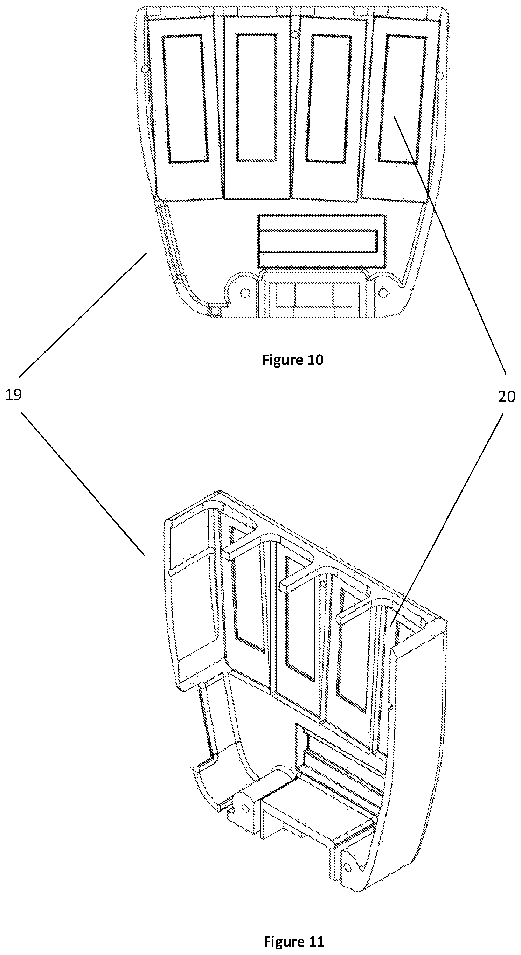

Fig. 10 demonstrates top view of palm cover according to the present invention.
Fig. 11 demonstrates isometric view of palm cover according to the present invention.

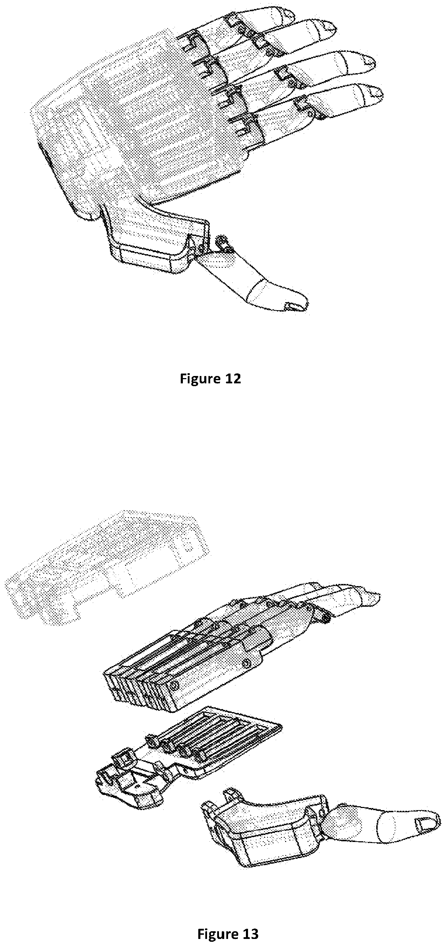

Fig. 12 demonstrates isometric view of prosthetic hand assembly according to the present invention.
Fig. 13 demonstrates exploded view of prosthetic hand assembly according to the present invention.

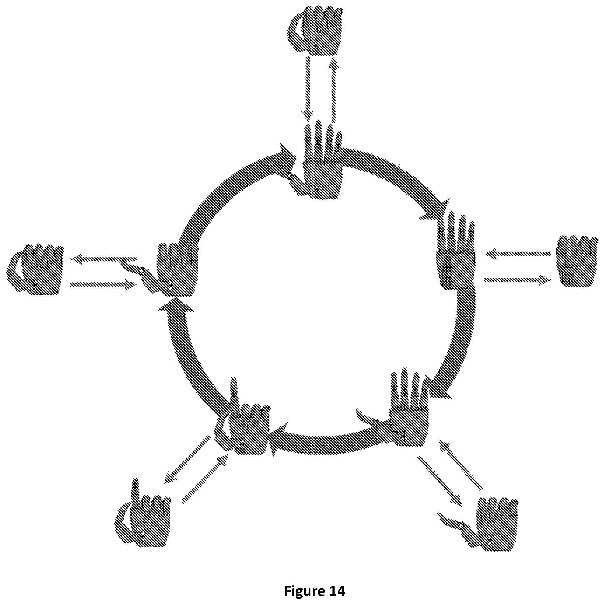

Fig. 14 demonstrates an exemplary finite state machine diagram of the prosthetic hand according to the present invention.

### Detailed Description of the Present Invention

The following numerals are referred to in the detailed description of the present invention:
- 1.: Finger member.
- 2.: Finger slider.
- 3.: Finger connecting link.
- 4.: Transmitting link.
- 5.: First group holes.
- 6.: Distal-intermediate phalange.
- 7.: Proximal phalange.
- 8.: Second group holes.
- 9.: Metacarpal.
- 10.: Thumb member.
- 11.: Thumb slider.
- 12.: Thumb connecting link.
- 13.: Thumb tip.
- 14.: Third group holes.
- 15.: Thumb base.
- 16.: Palm member.
- 17.: Opposability actuator.
- 18.: Opposability connecting link.
- 19.: Palm cover.
- 20.: Metacarpal receptacle.

The present invention discloses a prosthetic hand comprising finger members (1) and a thumb member (10) situated on a palm member (16) and covered with a palm cover (19) to encapsulate metacarpals (9) and opposability actuator (17), thus forming a hand. Metacarpals (9) and thumb base (15) which are positioned in allotted spaces, house actuators that enable the driving of respective finger (1) and thumb (10) members.

Finger member (1) includes three basic sub-members, designed and referred to regarding bone structure directly: A distal-intermediate phalange (6) along with preferably a contact force sensor therein (such as for instance as disclosed in WO2017200495) constitutes the outermost element, connected to a proximal phalange (7) through first group holes (5) to introduce a four-bar mechanism with consequently a single degree of freedom conjoined with a slider crank mechanism, details of which are to be further elaborated. Proximal phalange (7) is connected using second group holes (8) with the innermost element, a metacarpal (9), housing the motor actuating the finger member (1) in addition to itself being positioned inside the palm member (16). Said finger sub-members are connected by means of links (3, 4) and a finger slider (2) for the conclusive attachment of movable elements to said motor inside the metacarpal (9) for actuation thereof. To this extent, it can be restated that the slider crank mechanism of said finger member (1) is composed of said finger slider (2), finger connecting link (3) and shorter section of said transmitting link (4); while the four-bar mechanism of said finger member (1) is composed of said transmitting link (4), distal-intermediate phalange (6) andproximal phalange (7). Finger members (1) can further be of various sizes depending on the age of the human user, composed of appositely manufactured distal-intermediate (6), proximal (7) and metacarpal (9) phalanges.

Thumb member (10) comprises a thumb tip (13) yet still inspired by the relaxed state of lifelike distal and intermediate phalanges, a thumb base (15) and a motor for actuation thereof is housed therein. These two sub-members are connected at one end through third group holes (14), and at the other end via a thumb connecting link (12) forming a slider crank mechanism-utilizing movement scheme terminally along the thumb slider (11) which, driven by the motor situated within the thumb base (15), actuates the thumb member (10). To this extent, the slider crank mechanism of the thumb member (10) is composed of the thumb slider (11), thumb connecting link (12) and angled rear of the thumb tip (13). Similarly, thumb member (10) can be of various sizes depending on the age of the human user, composed of appositely manufactured thumb tip (13) and thumb base (15).

In order for the said thumb member (10) to oppose said finger members (1), its circumduction is externally driven using another motor, namely the opposability actuator (17) situated on a palm member (16) parallel to the wrist line, to which it is connected via the thumb base (15) using an opposability connecting link (18). Said palm member (16), in addition to accommodating said opposability actuator (17), possesses designated slots for the placement of motor-housing metacarpals (9) of the finger members (1).

A palm cover (19) with a housing for the opposability actuator (17) to be firmly stabilized, includes metacarpal receptacles (20) for individual finger members (1) as well. In total, five actuators are located when the palm member (16) is connected with the palm cover (19): four metacarpals (9) and one opposability actuator (17).

Movement-sustaining connection at finger members (1) are realized as follows: Angled rear end of the distal-intermediate phalanges (6), when attached to the transmitting link (4) constitutes for three of the bars of a four-bar mechanism, which defines two bars of said four-bar mechanism are included in said transmitting link (4) which is designed as a single part. Diagonal line of the proximal phalange (7) implicitly serves as the fourth and the final bar of the said four-bar mechanism. The relatively shorter extension of the transmitting link (4), also connected to the finger connecting link (3) associated with the finger slider (2) on the other end; serves as the common piece between the four-bar and the slider crank mechanism; as it also assumes the role of the outermost link of said slider crank mechanism composed via the association of finger slider (2) and the finger connecting link (3).

Finger member (1) actuators housed in metacarpals (9) drive the finger slider (2) towards the direction of the wrist and along with it the finger connecting link (3); inducing a rotative pull at the smallest bar of the four-bar mechanism, connected to the transmitting link (4). To the full extent this motion can go, it makes sure the distal-intermediate phalanges (6) are facing the wrist at the tip, closing the hand in the process of the motion. Same level of actuation may alternatively be attained with the aid of lead-screw mechanisms.

Movement-sustaining connection at thumb member (10) is similarly realized as follows: As previously stated, angled rear end of the thumb tip (13), together with the thumb connecting link (12) and the thumb slider (11) configure the slider crank mechanism to actuate the thumb member (10). Thumb member (10) actuator housed in thumb base (15) drives the thumb slider (11) towards the direction of the wrist and along with it the thumb connecting link (12); inducing a rotative pull and reversely, push directly concerning the angled rear of the said thumb tip (13), causing the to and fro movement thereof. To the full extent this motion can go, it makes sure the thumb tip (13) faces the finger members (1), enabling the various grip and hold motions of hand. Opposability of the thumb member (10) is extraneously ensured with the opposability actuator (17) in the palm member (16), structurally associated with the thumb base (15) via an opposability connecting link (18).

The hand in the present invention is capable of multiple grasps and hold formations as follows: In pinch grip mode, finger members (1) assuming roles of index and middle fingers fold, whereas the thumb member (10) is circumduced to oppose two finger members (1) while thumb member (10) simultaneously folds to accommodate an object such as a credit card. A similar derivative of said pinch grip mode includes only one finger member (1) assuming the role of the index finger and thumb member (10) opposing thereof, enabling the pinch of an item such as a key. Index pointer mode includes zero opposition of the thumb member (10), full extension of one finger member (1) assuming the role of index finger, and full curl of other finger members (1), enabling the wielder to point at surroundings with the index digit. Lateral grip mode includes zero opposition of the thumb member (10) along with full curl of all finger members (1) that can hold playing cards or pieces of board games such as draughts or backgammon. Power grip includes adaptive formation of individual finger members' (1) extensions, dictated by either a finite state machine or a machine learning-based control interface; that in turn enables the wielder to appropriately hold the object to be grasped, e.g. a mobile phone, a ball, a screwdriver. Circumduction of the thumb member (10) is also subject to the same interface still enabling the wielder to ascertain the desired position of the thumb member (10) in opposition mode.

Control and command of said finger member (1) and thumb members (10) are realized with the aforementioned finite state machine integrated with machine learning-based control schemes, e.g. pattern recognition: A finite state machine is a control flow that can assume only one of a given finite set of states at a given time, which, in the case of the present invention, pertains to the grips of the hand and individual opening-closing movements between. States in the present invention are divided into two sub-categories, namely open-hand and closed-hand, both of which include two further states for different grasp motions related thereto: Open hand has power grip and pinch grip pathways, whereas closed hand has pathways to index point and lateral grip, finalizing the total number of states at six.

In the case of a human with a transhumeral/transradial amputation wielding present invention, training is conducted utilizing electromyography (EMG) translating muscle contraction into electrical signals to be used within a simulation interface, through which said human assumes and develops control thereof. Electrodes are connected to both triceps and biceps, antagonism of which is deemed particularly advantageous in the sense that it can appropriately be projected on the relationship between open and close hand states, which are mutually exclusive. Bicep signal, tricep signal and a real-time modifiable threshold is introduced to ensure significant enablement of muscle movement to switch between different states.

Present invention, along with its previously stated design specifications ensures spatial efficiency especially at joint locations, which previously failed to allow individual electric motors to be situated therein due to their size. In devising a composition for joints which increases the effectiveness along with the utilization of said slider crank and four-bar mechanisms, present invention effectively surmounts the problem of inserting individual underactuation units thereon.

In a nutshell, the present invention proposes a prosthetic hand with thumb and finger members to be used by human amputees or robots in order to perform interactions with surroundings in addition to achieving most of the daily life activity grip and hold actions, while eliminating the necessity of inserting individual underactuation units and improving the space efficiency at the joints with said four-bar and slider crank mechanisms employed therein.

A prosthetic hand for humans and robots comprising multiple finger members (1) with connected distal-intermediate (6), proximal (7) and metacarpal phalanges (9) and a thumb member (10) with connected tip (13) and base (15); with said multiple finger members (1) and thumb member (10) accommodated in relation to a palm member (16) and a palm cover (19) constituting a digital multiplicity is proposed.

The finger members (1) each further comprise one finger connecting link (3) having a transmitting link (4) in the form of motion with conjoined four-bar and slider-crank mechanisms, connected to the actuator inside said metacarpal (9) via a finger slider (2) delivering the linear motion to excite the mechanisms for the actuation of said finger members (1).

The finger slider (2) member is a linear actuator, configured to drive a proximal (7) phalange member forwards and backwards for extension at the connecting link (3), thereby driving said transmitting link (4), said proximal (7) phalange member being the meeting point between said conjoined four-bar and slider-crank mechanisms.

The thumb base (15) of the thumb member (10) houses a motor for actuation thereof in accordance with said thumb slider (11) and thumb connecting link (12).

Opposability of said thumb member (10) is ensured by an opposability actuator (17).

The opposability actuator (17) is structurally associated with said thumb base (15) via an opposability connecting link (18).

The opposability actuator (17) is fixedly attached to the palm member (16).

In a further aspect of the present invention, said palm cover (19) comprises four metacarpal receptacles (20) for the fixed accommodation of metacarpals (9) of respective finger members (1).

In a further aspect of the present invention, the prosthetic hand employs a finite state machine for operation.

In a further aspect of the present invention, said finite state machine is trained with EMG input from a human user candidate.

## Claims

1. A prosthetic hand for humans and robots comprising multiple finger members (1) with connected distal-intermediate (6), proximal (7) and metacarpal phalanges (9) and a thumb member (10) with connected tip (13) and base (15); with said multiple finger members (1) and thumb member (10) accommodated in relation to a palm member (16) and a palm cover (19) constituting a digital multiplicity
said finger members (1) each further comprise one finger connecting link (3) having a transmitting link (4) in the form of motion with conjoined four-bar and slider-crank mechanisms, connected to the actuator inside said metacarpal (9) via a finger slider (2) delivering the linear motion to excite the mechanisms for the actuation of said finger members (1),
**characterized in that** said finger slider (2) being actuated via a linear actuator configured to drive a proximal (7) phalange member forwards and backwards for extension at the connecting link (3) thereby driving said transmitting link (4), said proximal (7) phalange member being the common member shared by said conjoined four-bar and slider-crank mechanisms,
the opposability of said thumb member (10) is extraneously ensured by an opposability actuator (17) fixedly attached to the palm member (16), said opposability actuator (17) being structurally associated with said thumb base (15) via an opposability connecting link (18), said thumb member comprising a thumb connecting link (12) and a thumb slider (11) together constituting motion with a slider-crank mechanism driven by a motor actuator housed by the thumb base (15) of said thumb member (10).

2. A prosthetic hand for humans and robots as set forth in Claim 1, **characterized in that** said palm cover (19) comprises four metacarpal receptacles (20) for the fixed accommodation of metacarpals (9) of respective finger members (1).

3. A prosthetic hand for humans and robots as set forth in any preceding Claim, **characterized in that** the prosthetic hand employs a finite state machine for operation.

4. A prosthetic hand for humans and robots as set forth in Claim 3, **characterized in that** said finite state machine is trained with EMG input from a human user candidate.

## Patentansprüche

1. Prothesenhand für Menschen und Roboter, die mehrere Fingerelemente (1) mit miteinander verbundenen End- Mittel-(6) Grund- (7) und Mittelhand-Gliedern (9) sowie ein Daumenelement (10) mit miteinander verbundener Spitze (13) und Basis (15) aufweist;
wobei die mehreren Fingerelemente (1) und das Daumenelement (10) in Bezug auf ein Handflächenglied (16) und eine Handflächenabdeckung (19) angeordnet sind und eine digitale Vielfältigkeit bilden
wobei jedes der Fingerelemente (1) ferner ein Fingerverbindungsgelenk (3) mit einem Übertragungsgelenk (4) mit verbundenen Viergelenk- und Schieber-Kurbel-Mechanismen für die Bewegung umfasst, die über einen Fingerschieber (2) mit dem Aktuator innerhalb des Mittelhandglieds (9) verbunden ist, der die lineare Bewegung zur Anregung der Mechanismen zur Betätigung der Fingerelemente (1) liefert, **dadurch gekennzeichnet, dass**
der Fingerschieber (2) über einen linearen Aktuator betätigt wird, der konfiguriert ist, um ein Grund-Gliedelement (7) vorwärts und rückwärts anzutreiben, um es am Verbindungsgelenk (3) zu strecken und dadurch das Übertragungsgelenk (4) anzutreiben, wobei das Grund-Gliedelement (7) das gemeinsame Element ist, das von den verbundenen Viergelenk- und Schieber-Kurbel-Mechanismen geteilt wird,
wobei die Opponierbarkeit des Daumenelements (10) durch einen externen, fest mit dem Handflächenglied (16) verbundenen Aktuator für die Opponierbarkeit (17) gewährleistet wird, wobei der Aktuator für die Opponierbarkeit (17) strukturell über ein Verbindungsglied für die Opponierbarkeit (18) mit der Daumenbasis (15) verbunden ist, wobei das Daumenelement ein Daumenverbindungsglied (12) und einen Daumenschieber (11) umfasst, die zusammen eine Bewegung mit einem Schieber-Kurbel-Mechanismus bilden, der von einem Motoraktuator angetrieben wird, der in der Daumenbasis (15) des Daumenelements (10) untergebracht ist.

2. Prothesenhand für Menschen und Roboter gemäß Anspruch 1, **dadurch gekennzeichnet, dass** die Handflächenabdeckung (19) vier Mittelhand-Aufnahmen (20) zur festen Aufnahme der Mittelhand-Glieder (9) der jeweiligen Fingerelemente (1) umfasst.

3. Prothesenhand für Menschen und Roboter gemäß irgendeinem der vorstehenden Ansprüche, **dadurch gekennzeichnet, dass** die Prothesenhand für den Betrieb einen endlichen Zustandsautomaten verwendet.

4. Prothesenhand für Menschen und Roboter gemäß Anspruch 3, **dadurch gekennzeichnet, dass** der endliche Zustandsautomat mit EMG-Eingaben von einem menschlichen Benutzerkandidaten trainiert wird.

## Revendications

1. Prothèse de main pour humains et robots comprenant plusieurs éléments formant doigts (1) avec des phalanges distales-intermédiaires (6), proximales (7) et métacarpiennes (9) reliées entre elles, et un élément formant pouce (10) avec une pointe (13) et une base (15) reliées entre elles ; lesdits multiples éléments formant doigts (1) et ledit élément formant pouce (10) étant logés par rapport à un élément formant paume (16) et un revêtement de paume (19) constituant une multiplicité digitale
lesdits éléments formant doigts (1) comprenant en outre chacun une articulation de connexion de doigt (3) comportant une articulation de transmission (4) sous la forme d'un mouvement avec des mécanismes à quatre barres et à manivelle-coulisseur conjoints, reliée à l'actionneur à l'intérieur dudit métacarpien (9) via un coulisseur de doigt (2) délivrant le mouvement linéaire pour exciter les mécanismes pour l'actionnement desdits éléments formant doigts (1), **caractérisée en ce que**
ledit coulisseur de doigt (2) est actionné via un actionneur linéaire configuré pour entraîner un élément de phalange proximale (7) vers l'avant et vers l'arrière pour l'extension au niveau de l'articulation de connexion (3), entraînant ainsi ladite articulation de transmission (4), ledit élément de phalange proximale (7) étant l'élément commun partagé par lesdits mécanismes à quatre barres et à manivelle-coulisseur conjoints,
l'opposabilité dudit élément formant pouce (10) est assurée de manière externe par un actionneur d'opposabilité (17) fixé de manière rigide à l'élément formant paume (16), ledit actionneur d'opposabilité (17) étant structurellement associé à ladite base de pouce (15) via une articulation de connexion d'opposabilité (18), ledit élément formant pouce comprenant une articulation de connexion de pouce (12) et un coulisseur de pouce (11) constituant ensemble un mouvement avec un mécanisme à coulisseur et manivelle entraîné par un actionneur à moteur logé dans la base de pouce (15) dudit élément formant pouce (10).

2. Prothèse de main pour humains et robots telle que décrite dans la revendication 1, **caractérisée en ce que** ledit revêtement de paume (19) comprend quatre réceptacles métacarpiennes (20) pour l'accommodation fixe des métacarpes (9) des éléments formant doigts (1) respectifs.

3. Prothèse de main pour humains et robots telle que décrite dans l'une quelconque des revendications précédentes, **caractérisée en ce que** la prothèse de main utilise une machine à états finis pour son fonctionnement.

4. Prothèse de main pour humains et robots telle que décrite dans la revendication 3, **caractérisée en ce que** ladite machine à états finis est entraînée à l'aide d'une entrée EMG provenant d'un utilisateur humain candidat.
